# EUROPEAN PATENT APPLICATION

(11) **EP 3 643 232 A1**
(43) Date of publication of application: **29.04.2020**
(21) Application number: 18201954.7
(22) Date of filing: 23.10.2018
(51) Int. Cl.: A61B 5/0488, A61B 5/11, A61B 5/00

(54) **APPARATUS AND METHOD FOR DETECTING NEUROLOGICAL DEFICITS**

(71) Applicant: Universitätsmedizin der Johannes Gutenberg-Universität Mainz, 55131 Mainz (DE)
(72) Inventor: Chaban, Ryan, 55131 Mainz (DE); Dohle, Daniel, 55131 Mainz (DE)
(74) Representative: Kuttenkeuler, David

(57) **Abstract**

An apparatus (1) and a method for monitoring neurological activities of a patient, in particular for detecting neurological deficits, which may occur for instance after surgical interventions in the descending aorta. An EMG signal (7) relating to electrical muscular activity of the patient is obtained by at least one electrode (2). The apparatus may be formed as a wearable device. The EMG signal is evaluated so as to detect a neurological deficit of the patient by determining a signal value from the EMG signal and classifying the signal value, wherein the EMG signal is associated with a neurological deficit if the signal value is below a predetermined threshold for a predetermined period of time.

## Description

### FIELD OF THE INVENTION

The present invention relates to an apparatus and a method for monitoring neurological activities of a bedridden patient, in particular for early detecting neurological deficits, which may occur for instance after surgical interventions in the descending aorta.

### BACKGROUND OF THE INVENTION

Recently, there is an increasing number of surgical interventions in the descending aorta due to more elderly patients and improved medical standards. However, these interventions may bear a risk of post-operative complications, such as spinal cord ischemia. Such ischemia caused by functional constriction or actual obstruction of blood vessels due to the use of stents may lead to edemas, which in turn may cause functional neurological deficits. While such deficits may be reversible if treated early, they may lead to severe irreversible damages such as paraplegia or incontinence. Similar neurological complications may occur also after other neurological interventions or strokes.

Irreversible damages may be prevented or at least reduced if the deficits are treated early. In particular, neurological deficits begin to occur within a critical time window of about 12 hours after the surgery and may be stopped and possibly reversed if detected early and treated immediately. Such neurological deficits may become apparent in reduced or missing ability of moving the extremities, which is usually monitored by medical staff who looks after the patient regularly, e.g. every two hours. However, e.g. due to hectic daily routine in clinics, subjective perception and occurrence of human errors, this method of monitoring neurological deficits is not reliable and error prone, and the abovementioned critical time window may pass by without taking any action. Also, the patient may still be immobile after anesthesia and may therefore not be able to show movements in his extremities, in particular legs.

### SUMMARY

It is therefore an object of the present invention to provide an apparatus and a method for easy, quick and reliable monitoring of neurological activities of a bedridden patient, in particular for early detecting neurological deficits.

This object is achieved according to the present invention by an apparatus and a method having the features of the independent claims. Preferred embodiments and further developments of the invention are specified in the claims dependent thereon.

According to the invention, an EMG signal is evaluated to detect a neurological deficit of the patient. The EMG signal may be obtained by means of at least one electrode, which obtains a signal relating to electrical muscular activity of the patient. EMG (electromyography) is a method of measuring electrical muscular activity. Where appropriate, at least two electrodes, such as two, three or four electrodes may be used. The at least one electrode may be applied to the patient's skin at a desired location, such as on the skin of one or both legs or of one or both arms of the patient. The EMG signal is evaluated by an evaluating unit, such as a microcontroller, so as to detect a neurological deficit of the patient. Upon evaluation of the EMG signal a signal value is determined from the EMG signal and the signal value is classified. The EMG signal is associated with a neurological deficit if the signal value is below a predetermined threshold for a predetermined period of time.

Thus, if the EMG signal, more specifically the determined signal value, is below a predetermined threshold for a predetermined period of time, this event is classified as a neurological inactivity, which may result from a neurological deficit. By detecting a neurological deficit by monitoring muscular activity by means of EMG as early as possible, actions can be taken to reverse the detected neurological deficit and to prevent or at least reduce further neurological deficits. The apparatus and method of the present invention provide an automated, easy and reliable way of detecting neurological deficits which may appear in bedridden patients, if applicable post-operatively bedridden patients, by eliminating subjective judgment by medical staff and eliminating human errors.

In order to classify the signal value to be related to electrical muscular activity or inactivity, the signal value may be associated with a value in a predetermined range, e.g. 0 to 100. If the value is above the predetermined threshold, which may be any appropriate value within the predetermined range, preferably close to the lower end, such as 15, it is considered as relating to activity, whereas it is considered as relating to inactivity if the value is below the predetermined threshold. If an inactivity is detected for a duration of at least the predetermined period of time, the evaluated signal is considered to indicate a neurological deficit. Inactivity for a shorter period of time than the predetermined period of time is considered as normal inactivity not related to a neurological deficit. For this purpose, a timer may be implemented which is restarted each time the signal value exceeds the predetermined threshold, e.g. by setting a timer counter to zero. The timer continues, i.e. the timer counter is increased, if the signal value is below the predetermined threshold. If the timer counter exceeds a predetermined timer threshold, a neurological deficit is assumed (watchdog timer).

Preferably, at least one of the predetermined threshold and the predetermined period of time is adjustable. The apparatus may comprise an adjustment device, which may include a mechanical potentiometer or other suitable adjusting knob or button. Likewise, the above parameters may be adjusted by means of an external unit connected e.g. wirelessly with the apparatus. By adjusting the threshold or the period of time or both the apparatus can be adjusted to the individual needs of a specific patient. For instance, some patients may have reduced muscular activity, which may require setting a lower threshold for the signal value than for other patients who have stronger muscular activity. This may reduce the number of false alarms. Likewise, the period of time during which an inactivity is monitored can be adjusted to determine when an inactivity is associated with a neurological deficit. For instance, for patients who tend to move their extremities less often, the period of time may be set to a higher value than for patients who tend to move more often so as to avoid any inactivity being associated with a neurological deficit. The predetermined period of time may be e.g. 10 minutes.

Before evaluating the EMG signal, the EMG signal may be processed, in particular to eliminate or at least reduce disturbances which do not relate to electrical muscular activity of the patient. Signal processing may include applying a filter on the EMG signal. Preferably, a filter is applied for eliminating mains hum (electric hum), which may be e.g. at a frequency of 50 Hz, 55 Hz, 60 Hz or 100 Hz depending on the local power-line frequency. Furthermore, a band-pass filter or band-stop filter may be applied. For instance, a band-pass filter that passes frequencies in the range of 70 to 100 Hz may be applied. It will be appreciated that other band-pass filters, band-stop filters, low-pass-filters or high-pass filters may be applied to enhance the signal quality so as to improve the evaluation result of the signal. This may improve the detection of neurological deficits. It will be appreciated that the signal processing may further include at least one of amplification (preferably before filtering) and boosting (preferably after filter) the signal to further improve the evaluation result. Furthermore, digital signal processing (DSP) may be performed on the EMG signal.

The EMG signal may be evaluated with respect to various parameters in order to detect an inactivity which indicates a neurological deficit. These parameters may include at least one of an amplitude, a frequency and a duration of a signal impulse of the EMG signal delivered by the at least one electrode. A respective signal value in the predetermined range as explained above may be determined for the respective parameter and then classified, i.e. compared with the predetermined threshold. Preferably, as also mentioned above, the EMG signal is evaluated after processing, in particular filtering, the EMG signal, which facilitates evaluation of the aforementioned parameters. The EMG signal may be evaluated continuously, which means that the electrodes continuously detect an input signal and the evaluation unit continuously evaluates the signal. A signal may be considered as being obtained "continuously" if the signal is obtained at a sufficiently high sample rate, e.g. at a rate of 500 Hz or more.

Preferably, once a neurological deficit is detected, an alarm signal is output. In other words, the apparatus may comprise an alarm unit for outputting an alarm signal, which may be triggered by the evaluating unit to output an alarm signal upon detection of a neurological deficit. The alarm signal may be any suitable signal, which can be acoustically, visually, haptically or otherwise perceived by the patient or another person, like medical staff or a doctor. Preferably, the alarm signal is an acoustic signal and the apparatus may comprise an acoustic unit for outputting the acoustic signal, such as a speaker. Alternatively or in addition, one or more LEDs for outputting a visual signal may be provided. Indicator LEDs having different colors may be provided to indicate the status of the apparatus, e.g. a general operating mode and an alarm mode. Further alternatively or additionally, the alarm signal may be output externally, such as on another mobile device or a stationary unit in the clinic to inform remote medical staff about an alarm. The connection with an external alarm unit may be wirelessly, e.g. via Bluetooth. An alarm as well as parts of or the entire EMG signal may be logged, i.e. stored in a memory, such as an SD card or other internal or external memory. An external memory may be connected wirelessly to the apparatus, e.g. via Bluetooth.

In some cases it may be advantageous if the patient is permitted to turn off the alarm without the intervention of medical staff by simply moving the monitored limb. For instance, if the apparatus detects a supposed neurological deficit due to inactivity of the patient, i.e. if the signal value is below the predetermined threshold for the predetermined period of time, although there is no neurological deficit, the patient may turn off the alarm by moving the respective extremity, thereby causing an electrical muscular signal, which is detected by the at least one electrode. The signal value will then exceed the predetermined threshold and the timer will be reset to zero as explained above. Possibly, a pre-alarm may be output to trigger the patient to move and to avoid an actual alarm, which would trigger medical staff to see the patient. In particular, the pre-alarm may include an alarm signal which is less noticeable than the actual alarm signal and which may be perceivable only for the patient, such as a low acoustic signal or a haptic signal, such as a vibration signal. For instance, a pre-alarm may be output if the signal value is below the predetermined threshold for a predetermined period of time, which is shorter than the predetermined period of time defining an actual neurological deficit. In this way, the apparatus will serve as a reminder for the patient to perform periodical self-checking. In other words, the apparatus may not only function as an alarm device for the medical staff but may actively trigger the patient to move.

Preferably, the apparatus of the present invention is a wearable device, which may be battery powered or powered by a rechargeable battery. A wearable device is a device which can be worn by the patient, attached directly to the patient's body or embedded in clothes. The wearable device may be a wearable band, such as a wristband or other band which may be worn at a target location on any extremity, such as an ankle of a leg, or may be any other suitable wearable device, like a clip, sticker, or the like. The at least one electrode may be placed on a skin facing surface of the wearable device, e.g. the inner side of a wristband, such that in use the at least one electrode contacts the patient's skin. Thus, the apparatus of the present invention may be of a small size, easy to handle and convenient to wear for the patient. Preferably, no additional clinical infrastructure is necessary. It will be appreciated, however, that the apparatus may be included in a system and may transmit data, preferably wirelessly, such a via Bluetooth, to another mobile device or stationary unit, for instance to inform remote medical staff in case of an alarm or to store the signal data. In any case, the present invention provides an easy, quick and reliable method of early detecting neurological deficits, thereby permitting prevention or at least reduction of long-term damages.

### BRIEF DESCRIPTION OF THE DRAWINGS

The foregoing summary, as well as the following detailed description of preferred embodiment, will be better understood when read in conjunction with the appended drawings. For the purpose of illustrating the present disclosure, reference is made to the drawings. The scope of the disclosure is not limited, however, to the specific embodiment disclosed in the drawings. In the drawings:
Fig. 1 shows a wearable device according to the invention.
Fig. 2 schematically shows the components of the device of Fig. 1.
Fig. 3 shows an EMG signal before and after filtering.

### DETAILED DESCRIPTION

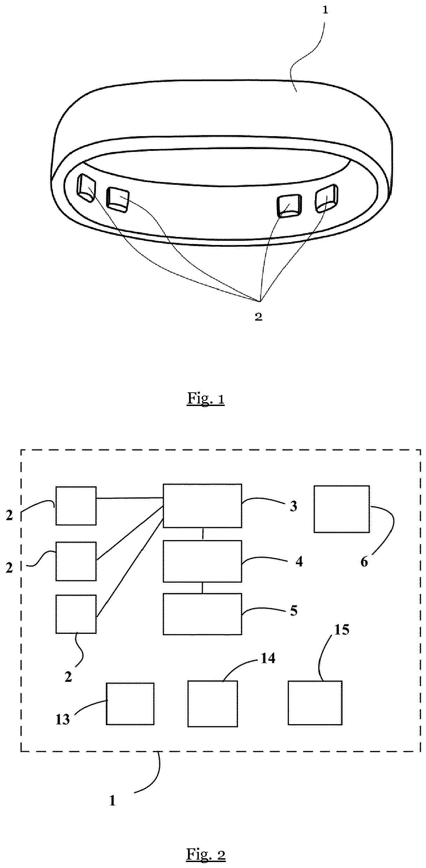

Referring to Fig. 1, an apparatus in accordance with an embodiment of the present invention in the form of a wearable device 1 is shown. In the embodiment shown, the wearable device 1 is a wristband, which can be worn by a patient around a wrist in order to monitor muscular activity of the respective arm. Alternatively, in order to monitor muscular activity of a leg, a wearable device may be formed as a band for wearing around the ankle of the respective leg. As will be explained in more detail below, the wearable device 1 includes at least one electrode 2, such as four electrodes 2 in the embodiment shown in Fig. 1, to detect an EMG signal when in contact with the patient's skin. As will be appreciated by a person skilled in the art, EMG (electromyography) is a method of measuring electrical muscular activity of a patient. The EMG signal may be used to detect electrical muscular inactivity, which may result from a neurological deficit. The wearable device 1 is able to give an alarm if inactivity relating to a neurological deficit is detected as will be explained in more detail below, thereby permitting immediate treatment of a supposed neurological defect.

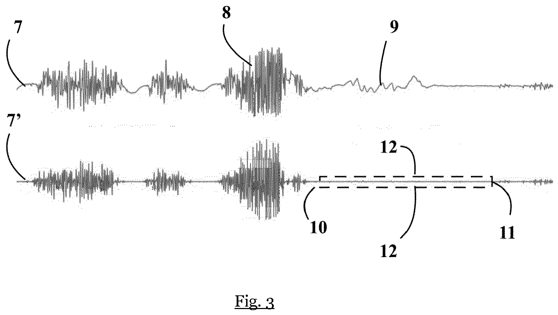

The configuration of the wearable device 1 is schematically illustrated in Fig. 2 to explain the components and their relationship. It will be appreciated that further components may be included in the device to add further functionality or comfort, or some components may be omitted. As mentioned above, the device 1 includes at least one electrode 2 for detecting an EMG signal (see Fig. 3), i.e. a signal relating to electrical muscular activity of the patient, when in contact with the patient's skin. The electrodes 2 are disposed on a skin facing surface of the wearable device 1, i.e. on an inner side of the wristband, and may be arranged along the length of the device, i.e. around the circumference of the patient's extremity, in any suitable configuration to detect the EMG signal.

The EMG signal is processed by a signal processing unit 3 to enhance the signal quality and remove possible disturbances which do not relate to muscular activity of the patient, such as e.g. mains hum. The signal processing unit 3 may perform signal amplification, filtering, boosting, and digital signal processing as necessary and desired. For instance, an appropriate band-pass filter or low-pass filter may be applied to eliminate undesired frequency portions.

Fig. 3 in the upper portion shows the actual EMG signal 7 obtained by the electrodes 2, whereas the lower portion in Fig. 3 illustrates the processed EMG signal 7'. Disturbances 9 are eliminated, while the actual signal amplitude 8 indicating muscular activity is maintained and possibly enhanced. Hereinafter, reference to the EMG signal 7 will particularly include reference to the processed EMG signal 7'.

Still referring to Fig. 2, the wearable device 1 further includes an evaluation unit 4 for evaluating the EMG signal 7 (more specifically the processed EMG signal 7') so as to detect neurological deficits of the patient, as will be explained in more detail below. If a neurological deficit is detected, the evaluation unit 4 triggers an alarm unit 5 to output an alarm signal, which may be an acoustic signal, such as a beep. The alarm signal may be output directly by the wearable device 1 and/or may be sent to an external unit (not shown) via a wireless interface 6, such as via Bluetooth. The wearable device 1 is battery powered by means of a battery 13, which may be a rechargeable battery if desired. An alarm event, any other event or the EMG signal as such may be logged and stored in a memory 14, such as an SD card or an extern memory (not shown).

Referring again to Fig. 3, a method of evaluating the EMG signal 7 in order to detect neurological deficits of a patent is explained in more detail. The method may be implemented as a software program running on the evaluation unit 4, which is preferably a microcontroller. It will be appreciated that the raw EMG signal 7 may be evaluated. However, as explained above, the EMG signal 7 will be processed, in particular amplified and filtered, to obtain the processed EMG signal 7', which will improve reliability of the method. Evaluating the EMG signal 7 (more specifically the processed EMG signal 7') includes determining a signal value from the EMG signal 7. This may include determining a value in a predetermined range, such as 0 to 100, wherein the limits of this range may be associated with possible minimum and maximum values. The EMG signal 7 may be evaluated with respect to at least one of an amplitude, a frequency and a duration of a signal impulse.

The determined value in the aforementioned range is then classified, which means that it is compared to a predetermined threshold. The predetermined threshold may be set in accordance with an activity level of the patient. Preferably, the predetermined threshold is closer to the lower end of the range to permit detecting inactivity. For instance, in the aforementioned range of 0 to 100, the predetermined threshold may be e.g. 15. In particular, the threshold may be adjusted by means of an adjustment device 15 (see Fig. 2). Likewise, the predetermined period of time (see below) may be adjustable by the same or another adjustment device 15, which may be internal or external to the wearable device 1.

For illustrative purposes only, the predetermined threshold is indicated in Fig. 3 by means of dashed line 12. However, as explained above, a determined signal value which relates to at least one of an amplitude, frequency or duration of a signal impulse will actually be compared to the threshold value. Since inactivity of the patient for a short period of time does not necessarily relate to a neurological deficit, a neurological deficit is indicated only when the signal value is below the predetermined threshold for a predetermined period of time. The beginning 10 and end 11 of the predetermined period of time are indicated in Fig. 3 for illustrative purposes. A timer or timer counter may be reset each time the signal value exceeds the predetermined threshold. Once the timer counter reaches or exceeds a predetermined timer threshold, the alarm unit is triggered, and an alarm signal is output as explained above.

It will be appreciated that the above description of the preferred embodiment is not meant to be limiting the scope of the present invention. In particular, more or less electrodes may be used to obtain the EMG signal. The wireless interface may be omitted if no data transmission is envisioned. Alternatively or additionally, a cable connection with an external unit may be possible. If no data are to be logged, the memory unit may be omitted. Further, the processing unit, evaluation unit and alarm unit may be separate units or combined as desired in one or more integrated units. Furthermore, the values given in the method of evaluating the EMG signal are only exemplary and may be chosen as desired. The adjustment unit may be omitted if the method is intended to be carried out using fixed values. Likewise, the parameters of the method could be adjusted by means of an external unit, such as another mobile device, connected wirelessly to the apparatus of the present invention. For instance, an external control unit could be envisioned to configure and control operation of the wearable device. While the above embodiment is described with respect to a wearable wristband, any other configuration, in particular wearable configuration, of the apparatus of the present invention may be envisioned as desired.

## Claims

1. An apparatus (1) for monitoring neurological activities of a bedridden patient, comprising:
at least one electrode (2) for obtaining an EMG signal (7) relating to electrical muscular activity of the patient; and
an evaluating unit (4) for evaluating the EMG signal, the evaluating unit (4) configured to detect a neurological deficit of the patient by determining a signal value from the EMG signal and classifying the signal value, wherein the EMG signal is associated with a neurological deficit if the signal value is below a predetermined threshold for a predetermined period of time.

2. The apparatus of claim 1, further comprising a processing unit (3) for processing the EMG signal before evaluating the EMG signal, the processing unit (3) configured to apply a filter on the EMG signal so as to eliminate disturbances which do not relate to electrical muscular activity of the patient.

3. The apparatus of claim 1 or 2, further comprising an alarm unit (5) for outputting an alarm signal, wherein the evaluating unit (4) is configured to trigger the alarm unit (5) to output an alarm signal upon detection of a neurological deficit.

4. The apparatus of any one of claims 1 to 3, wherein the apparatus (1) comprises a wearable device, which is preferably battery powered.

5. A method of monitoring neurological activities of a bedridden patient, comprising the steps of:
obtaining an EMG signal relating to electrical muscular activity of the patient; and
evaluating the EMG signal so as to detect a neurological deficit of the patient by determining a signal value from the EMG signal and classifying the signal value, wherein the EMG signal is associated with a neurological deficit if the signal value is below a predetermined threshold for a predetermined period of time.

6. The method of claim 5, further comprising, before the step of evaluating the EMG signal, a step of processing the EMG signal including applying a filter on the EMG signal so as to eliminate disturbances which do not relate to electrical muscular activity of the patient.

7. The method of claim 5 or 6, wherein at least one of the predetermined threshold and the predetermined period of time is adjustable.

8. The method of any one of claims 5 to 7, wherein the step of evaluating the EMG signal comprises determining the signal value with respect to at least one of an amplitude, a frequency and a duration of a signal impulse of the EMG signal.

9. The method of any one of claims 5 to 8, further comprising the step of outputting an alarm signal when a neurological deficit is detected.

10. The apparatus of any one of claims 1 to 4, configured to carry out the method of any one of claims 5 to 9.
